# EUROPÄISCHE PATENTSCHRIFT

(11) **EP 3 713 466 B1**
(45) Veröffentlichungstag und Bekanntmachung des Hinweises auf die Patenterteilung: **31.05.2023**
(21) Anmeldenummer: 18800919.5
(22) Anmeldetag: 09.11.2018
(51) Int. Cl.: A61B 1/00, A61B 1/12, A61B 90/70, A61L 2/26, G02B 27/01

(54) **BENUTZERASSISTENZSYSTEM FÜR WIEDERVERWENDBARE MEDIZINPRODUKTE**
USER ASSISTANCE SYSTEM FOR REUSABLE MEDICAL PRODUCTS
SYSTÈME D'ASSISTANCE À UN UTILISATEUR POUR DES PRODUITS MÉDICAUX RÉUTILISABLES

(30) Priorität: 23.11.2017 DE 102017127718
(43) Veröffentlichungstag der Anmeldung: 30.09.2020
(73) Patentinhaber: Olympus Winter & Ibe GmbH, 22045 Hamburg (DE)
(72) Erfinder: JÜRGENS, Thorsten, 21037 Hamburg (DE); ROSENKRANZ, Kristin, 21220 Seevetal (DE)
(74) Vertreter: Seemann & Partner Patentanwälte mbB
(86) Internationale Anmeldenummer: PCT/EP2018/080757
(87) Internationale Veröffentlichungsnummer: WO 2019/101545

(56) Entgegenhaltungen:
- WO-A1-2015/175681
- DE-A1-102014 217 559
- JP-A- 2009 291 308

## Beschreibung

Die Erfindung betrifft ein Benutzerassistenzsystem für wiederverwendbare Medizinprodukte, ein Verfahren zum Überwachen der Aufbereitung von wiederverwendbaren Medizinprodukten sowie ein Computerprogramm.

Wiederverwendbare Medizinprodukte, wie beispielsweise Endoskope, müssen aus hygienischen Gründen nach ihrer Benutzung aufbereitet werden. Zu diesem Zweck stellt der Hersteller der wiederverwendbaren Medizinprodukte den Anwendern Aufbereitungsinformationen zur Verfügung, die die erforderlichen Anweisungen und Hinweise zur erfolgreichen Aufbereitung der Medizinprodukte enthalten.

Vielfach liegen diese Aufbereitungsinformationen als Druckerzeugnis vor, beispielsweise als gedruckte Gebrauchsanweisung oder als Aufbereitungskarte. Teilweise werden die Aufbereitungsinformationen auch online bereitgestellt.

Für jeden Typ von wiederverwendbarem Medizinprodukt müssen spezifische Aufbereitungsinformationen bereitgestellt werden, was zu einer großen Anzahl an Gebrauchsanweisungen führt, die permanent in Benutzung sind.

Liegt die Gebrauchsanleitung beispielsweise als Druckerzeugnis vor, braucht der Anwender zur Handhabung wenigstens eine freie Hand. Auch wenn die Gebrauchsanweisung beispielsweise auf einem Tablet gespeichert ist, vereinfacht sich die Handhabung nicht wesentlich, da der Anwender in diesem Fall das Tablet bedienen muss. In beiden Fällen ist es für den Anwender schwierig, gleichzeitig die Anweisungen zu lesen und diese auszuführen. Dies verkompliziert die Aufbereitung und die Anweisungen werden eventuell nicht korrekt umgesetzt.

Es sind Benutzerassistenzsysteme bekannt, bei denen das Personal mittels einer Datenbrille bei der Aufbereitung unterstützt wird. Ein solches Benutzerassistenzsystem ist beispielsweise in der DE 10 2014 217 559 A1 offenbart. Bei diesem System wird mittels einer in einer Datenbrille eingebauten Kamera ein Identifikationsmerkmal eines bestimmten Typs von chirurgischem Instrument erfasst. Die erzeugten Bilddaten werden mit auf einem Datenspeicher gespeicherten Identifikationsmerkmalen verglichen und bei Übereinstimmung werden visuelle Informationen betreffend Anweisungen zur Reinigung dieses speziellen Typs von chirurgischem Instrument auf einer Projektionsfläche der Datenbrille angezeigt. Der Anwender hat bei der Bedienung des Systems beide Hände frei, um die Anweisungen direkt umzusetzen. Zudem ist es nicht notwendig, eine spezifische Gebrauchsanweisung herauszusuchen.

Aus WO 2015/175681 A1 ist eine Wiedergabevorrichtung bekannt, welche auf dem Kopf getragen wird und eine Wiedergabeeinheit und eine Schnittstelle zu einem medizinischen Gerät aufweist. Die Vorrichtung ist dazu eingerichtet, während einer invasiven Behandlung eines Patienten getragen zu werden. Sie kommuniziert drahtlos mit einem Netzwerk und weist eine Verarbeitungseinheit auf, die Daten von dem medizinischen Gerät empfängt. Die Verarbeitungseinheit ruft aus einem Speicher Anweisungen betreffend das medizinische Gerät ab, welche auf den von dem medizinischen Gerät empfangenen Daten basieren. Die Anweisungen betreffend das medizinische Gerät werden auf einem Bildschirm des auf dem Kopf getragenen Geräts wiedergegeben.

Es ist eine Aufgabe der Erfindung, ein Benutzerassistenzsystem für wiederverwendbare Medizinprodukte und ein Verfahren zum Überwachen der Aufbereitung von wiederverwendbaren Medizinprodukten anzugeben, mit dem eine schnelle und sichere Aufbereitung von wiederverwendbaren Medizinprodukten erreicht werden kann.

Die Aufgabe wird gelöst durch ein Benutzerassistenzsystem für wiederverwendbare Medizinprodukte, umfassend eine Datenbrille mit einer Kamera, einem bildgebenden optischen Modul mit einer Projektionsfläche, wobei das Benutzerassistenzsystem ferner einen Datenspeicher umfasst, auf dem eine Vielzahl von Typdatensätzen, jeweils umfassend Informationen betreffend ein Identifikationsmerkmal eines bestimmten Medizinprodukt-Typs, und Informationen betreffend Anweisungen zur Wiederaufbereitung dieser Medizinprodukt-Typen gespeichert sind, wobei
- das bildgebende optische Modul dazu eingerichtet ist, visuelle Informationen auf die im Blickfeld eines Nutzers der Datenbrille angeordnete Projektionsfläche zu projizieren,
- das Benutzerassistenzsystem dazu eingerichtet ist, ein Bild eines wiederverwendbaren Medizinprodukts mit der Kamera zu erfassen, die Bilddaten des Bilds mit den Informationen betreffend die Identifikationsmerkmale in den Typdatensätzen abzugleichen, einen Typ des wiederverwendbaren Medizinprodukts anhand eines in dem Bild vorhandenen Identifikationsmerkmals zu identifizieren und ein einem zugehörigen Typdatensatz zugeordnetes Typprofil zu aktivieren,
   wobei das Benutzerassistenzsystem ferner dazu eingerichtet ist,
- ein Nutzeridentifikationsmerkmal zu erfassen und mit auf dem Datenspeicher gespeicherten Nutzerdatensätzen, die jeweils Informationen betreffend wenigstens ein Nutzeridentifikationsmerkmal umfassen, abzugleichen und bei Übereinstimmung ein dem Nutzerdatensatz zugeordnetes Nutzerprofil zu aktivieren,
- ein Standortidentifikationsmerkmal zu erfassen und mit auf dem Datenspeicher gespeicherten Standortdatensätzen, die jeweils Informationen betreffend wenigstens ein Standortidentifikationsmerkmal umfassen, abzugleichen und bei Übereinstimmung ein dem Standortdatensatz zugeordnetes Standortprofil zu aktivieren,
- wobei der Datenspeicher Informationen betreffend Anweisungen zur Wiederaufbereitung von Medizinprodukten umfasst, die zumindest einem Typprofil, zumindest einem Nutzerprofil und zumindest einem Standortprofil zugeordnet sind, und wobei die dem aktiven Typprofil, dem aktiven Nutzerprofil und dem aktiven Standortprofil zugeordneten Anweisungen zur Wiederaufbereitung des Medizinprodukts als visuelle Informationen auf der Projektionsfläche angezeigt werden.

Unter dem Begriff "Anweisungen" werden im Kontext der vorliegenden Beschreibung Handlungsanweisungen verstanden, die dem Anwender mittels auf der Projektionsfläche eingeblendeter Texte, Bilder, Videos oder ähnlichem bei der Aufbereitung des Medizinprodukts unterstützen. Bei den Anweisungen kann es sich beispielsweise um Hinweise oder Warnhinweise handeln.

Unter einem "Typprofil" werden Informationen zur Konfiguration des Benutzerassistenzsystems verstanden. Bei Aktivierung des Typprofils wird das Benutzerassistenzsystem gemäß diesen Informationen konfiguriert. Die Begriffe Nutzerprofil und Standortprofil sind analog zu verstehen.

Vorteilhaft bietet das erfindungsgemäße Benutzerassistenzsystem eine bessere Handhabbarkeit als Gebrauchsanweisungen. Weiterhin vorteilhaft werden durch das erfindungsgemäße Benutzersystem nur solche visuellen Informationen betreffend Anweisungen zur Aufbereitung eines wiederverwendbaren Medizinprodukts auf der Projektionsfläche der Datenbrille angezeigt, die im Kontext mit dem Medizinprodukt-Typ, dem Nutzer der Datenbrille und dem Standort, an dem sich der Nutzer aufhält, stehen. So sind die angezeigten Informationen zunächst davon abhängig, welcher Typ von wiederverwendbarem Medizinprodukt aufbereitet werden soll. Zusätzlich sind die angezeigten Informationen aber auch davon abhängig, welcher Nutzer die Datenbrille trägt. Ein Mitarbeiter, der beispielsweise für die Sterilisation eines Medizinprodukts verantwortlich ist, bekommt ausschließlich Informationen angezeigt, die für die Sterilisation des Medizinprodukts von Bedeutung sind. Informationen, die hingegen nur für einen Mitarbeiter von Bedeutung sind, der Medizinprodukte im Operationssaal für die Nutzung vorbereitet, werden hingegen nicht eingeblendet. Auf diese Weise werden keine überflüssigen Informationen im Sichtfeld des Anwenders eingeblendet, so dass dieser nicht erst die für ihn relevanten Informationen heraussuchen muss.

Um die angezeigten Informationen noch stärker auf die tatsächlich relevanten Informationen zu beschränken, wird zusätzlich die Erkennung des derzeitigen Standorts mittels der Standortidentifikationsmerkmale durchgeführt. Die auf der Projektionsfläche der Datenbrille angezeigten Informationen sind also vom Kontext des derzeitigen Standorts abhängig. So werden beispielsweise an einem Spülbecken andere Informationen eingeblendet als an einer Aufbereitungsmaschine.

Vorzugsweise werden keine Informationen eingeblendet, wenn kein Typprofil oder kein Nutzerprofil aktiviert sind. Dies beugt der unbefugten Nutzung des Systems vor. Es kann aber auch vorgesehen sein, dass bestimmte Informationen unabhängig von dem aktiven Typ-, Nutzer- oder Standortprofil immer eingeblendet werden, beispielsweise Warnhinweise.

In einer Ausführungsform ist der Datenspeicher im Gehäuse der Datenbrille angeordnet. In einer anderen Ausführungsform ist der Datenspeicher beispielsweise in einem separaten Server angeordnet, der über eine drahtlose Datenverbindung mit der Datenbrille verbunden ist. Ein solcher separater Server ist beispielsweise in DE 10 2014 217 559 A1 beschrieben.

Vorzugsweise ist das Standortidentifikationsmerkmal in einem RFID- oder NFC-Transponder gespeichert, in einem Barcode oder einem DataMatrix-Code codiert und/oder aus einer äußeren Erscheinung zumindest eines ortsfesten Gegenstands bestehend.

Für den Fall, dass wenigstens ein Standortidentifikationsmerkmal in einem RFID- oder NFC-Transponder gespeichert ist, weist die Datenbrille einen Empfänger zum Empfang von RFID- oder NFC-Signalen auf. Das Standortidentifikationsmerkmal ist insbesondere eine maschinenlesbare Codierung. Es handelt sich beispielsweise um eine lineare Codierung (z.B. GS1-128 Barcode), eine zweidimensionale Codierung (z.B. Data Matrix-Code) oder auch um eine dreidimensionale Codierung. Ebenso können Symbole und/oder Schriftzeichen eingesetzt werden. Ist wenigstens ein Standortidentifikationsmerkmal beispielsweise in einem Barcode oder einem DataMatrix-Code (QR-Code) codiert, so ist die Kamera zur Erfassung und das Benutzerassistenzsystem zur Auswertung dieser Codes eingerichtet. Besteht wenigstens ein Standortidentifikationsmerkmal aus einer äußeren Erscheinung zumindest eines ortsfesten Gegenstands, so ist die Kamera zur Erfassung eines Bildes dieses zumindest einen ortsfesten Gegenstands und das Benutzerassistenzsystem zur Analyse der Bilddaten dieses Bildes eingerichtet. Zur Analyse kommen dabei vorzugsweise bekannte Techniken der Bildanalyse zum Einsatz.

Vorzugsweise ist das Nutzeridentifikationsmerkmal ein Passwort und/oder ein biometrisches Merkmal des Nutzers. Das Passwort kann beispielsweise manuell über eine Benutzeroberfläche an der Datenbrille eingegeben werden und/oder es kann sich um ein gesprochenes Wort handeln. Im letzteren Fall ist die Datenbrille mit einem Mikrofon zur Erfassung der akustischen Signale ausgestattet und das Benutzerassistenzsystem zur Spracherkennung durch Analyse der erfassten akustischen Signale eingerichtet. Bei dem biometrischen Merkmal kann es sich beispielsweise um einen Fingerabdruck, ein Irismuster, ein Netzhautmuster oder die Stimme eines Nutzers handeln. Zur Erkennung des Fingerabdrucks ist das Benutzerassistenzsystem mit einer Vorrichtung zur Erfassung eines Fingerabdrucks eingerichtet, umfasst also beispielsweise einen Fingerabdruckscanner. Zur Erfassung eines biometrischen Merkmals im Augenbereich des Nutzers ist die Datenbrille beispielsweise mit wenigstens einer zusätzlichen Kamera ausgestattet, die zur Erfassung eines Bildes des Augenbereichs des Nutzers eingerichtet ist, während dieser die Datenbrille verwendet. Alternativ kann auch nur eine Kamera vorgesehen sein, die sowohl den Augenbereich des Nutzers als auch den Bereich des Blickfelds des Nutzers erfassen kann. Zur Erkennung der Stimme ist ein Mikrofon am Gehäuse der Datenbrille vorgesehen. Anders als bei dem gesprochenen Passwort ist das Benutzerassistenzsystem in diesem Fall jedoch nicht zur Durchführung einer Spracherkennung, sondern zur Durchführung einer Stimmerkennung eingerichtet. Besonders bevorzugt ist das Benutzerassistenzsystem sowohl zur Durchführung einer Spracherkennung als auch einer Stimmerkennung eingerichtet.

Gemäß einer weiteren Ausführungsform ist das Benutzerassistenzsystem dazu eingerichtet, Anweisungen betreffend die Wiederaufbereitung des Medizinprodukts als visuelle Informationen auszugeben und durch Auswertung der von der Kamera aufgenommenen Bilddaten eine Ausführung dieser Anweisung zu überwachen, wobei insbesondere bei fehlerhafter Ausführung der Anweisung das Benutzerassistenzsystem zur Ausgabe einer Fehlermeldung eingerichtet ist.

Eine solche Anweisung kann beispielsweise die Reinigung des Medizinprodukts mit einer Bürste betreffen. Beispielsweise muss ein bestimmter Kanal eines Endoskops 10-mal gebürstet werden. Durch die Erfassung der Bilddaten mit der Kamera und Auswertung dieser Bilddaten erkennt das Benutzerassistenzsystem, ob diese Anweisung korrekt ausgeführt wurde. Wird beispielsweise nur 9-mal gebürstet, so gibt das Benutzerassistenzsystem eine Fehlermeldung auf der Projektionsfläche der Datenbrille aus.

Bei der Anweisung kann es sich aber auch um Informationen betreffend die korrekte Zuordnung der entsprechenden Anschlüsse an einem Endoskop in einer Endoskop-Aufbereitungsvorrichtung handeln. Das Benutzerassistenzsystem überprüft in diesem Fall, ob alle Anschlüsse korrekt angeschlossen wurden.

Gemäß einer vorteilhaften Ausführungsform umfasst das Benutzerassistenzsystem eine Lichtquelle, welche in einem Wellenlängenbereich emittiert, in dem medizinische Verunreinigungen, insbesondere Körperflüssigkeiten, die Proteine enthalten, wie z.B. Blut oder Schleim, zu Fluoreszenz angeregt werden, wobei die Kamera zur Bilderfassung in einem Wellenlängenbereich eingerichtet ist, in dem die Fluoreszenz der medizinischen Verunreinigungen auftritt, wobei das Benutzerassistenzsystem ferner zur Erzeugung von Fluoreszenzbildern, insbesondere zur Verstärkung von Fluoreszenzbildern, und zur Darstellung derselben auf der Projektionsfläche eingerichtet ist.

Vorteilhaft wird hierdurch eine bessere Erfolgskontrolle der Aufbereitung erreicht, weil Körperflüssigkeiten, die Proteine enthalten (wie z.B. Blut oder Schleim) sichtbar gemacht werden, die ansonsten möglicherweise nicht erkennbar gewesen wären. Auf diese Weise kann überprüft werden, ob die Aufbereitung eines wiederverwendbaren Medizinprodukts erfolgreich durchgeführt wurde.

Vorteilhaft ist die Navigation in den visuellen Informationen, die auf der Projektionsfläche eingeblendet werden, durch Erkennung von Augenbewegungen, Gestenerkennung, Spracherkennung und/oder mechanische Betätigung eines Eingabeelements am Gehäuse der Datenbrille steuerbar.

Insbesondere umfasst die Navigation in den visuellen Informationen das Umblättern von Seiten, die Auswahl von Informationselementen oder das Bestätigen von Eingaben. Zur Erkennung der Augenbewegungen umfasst das Benutzerassistenzsystem beispielsweise eine der zuvor beschriebenen Kameras zur Erfassung des Augenbereichs des Nutzers. Zudem ist es dazu eingerichtet, die erfassten Bilddaten zu analysieren und beispielsweise zu bestimmen, wohin der Nutzer blickt. Zur Gestenerkennung ist das Benutzerassistenzsystem zur Analyse der von der Kamera erfassten Bilddaten eingerichtet, wobei die Bilddaten beispielsweise mit zuvor auf dem Datenspeicher gespeicherten Gestenbewegungen verglichen werden. Dadurch wird es beispielsweise möglich, mittels einer Winkbewegung die auf der Projektionsfläche eingeblendeten Informationen zu verändern, beispielsweise von einer Seite zur nächsten zu blättern.

Gemäß einer weiteren Ausführungsform ist das Benutzerassistenzsystem zur Erfassung und Dokumentation der laufenden und abgeschlossenen Wiederaufbereitungsvorgänge eingerichtet, wobei das Benutzerassistenzsystem ferner einen externen Datenspeicher umfasst, der zur Speicherung der Informationen betreffend die laufenden und abgeschlossenen Wiederaufbereitungsvorgänge eingerichtet ist.

Die Informationen betreffen die laufenden und abgeschlossenen Wiederaufbereitungsvorgänge, umfassen insbesondere Informationen über den Status des Wiederaufbereitungsvorgangs, also ob dieser abgeschlossen ist, gerade durchgeführt wird oder noch nicht begonnen hat. Zudem umfassen die Informationen vorzugsweise auch den Zeitpunkt des Beginns und des Endes des Wiederaufbereitungsvorgangs, den Typ des Medizinprodukts, den Nutzer, der den Wiederaufbereitungsvorgang durchführt, den Standort und/oder Informationen darüber, um welchen Wiederaufbereitungsschritt es sich handelt.

Bei dem externen Datenspeicher kann es sich um denselben Datenspeicher handeln, auf dem auch die Anweisung zur Aufbereitung der Medizinprodukte gespeichert ist, sofern es sich bei letzterem ebenfalls um einen externen Datenspeicher handelt. Es kann sich aber auch um zwei getrennte Datenspeicher handeln. Insbesondere wird durch die Speicherung der Dokumentation auf einem externen Datenspeicher die Sicherheit der Aufbereitung erhöht, da die Informationen außerhalb des direkten Zugriffs des Nutzers gespeichert werden. Vorzugsweise ist ebenfalls vorgesehen, dass die auf dem externen Datenspeicher gespeicherten Informationen jederzeit von wenigstens einem dazu eingerichteten Arbeitsplatz abrufbar sind. Dadurch kann von diesem Arbeitsplatz aus die Aufbereitung eines wiederverwendbaren Medizinprodukts jederzeit nachvollzogen werden.

Gemäß einer weiteren vorteilhaften Ausführungsform ist das Benutzerassistenzsystem dazu eingerichtet, wenigstens einem Wiederaufbereitungsvorgang eine vorgesehene Aufbereitungszeit zuzuordnen, und auf der Projektionsfläche eine Restdauer der vorgesehenen Aufbereitungszeit anzuzeigen, wobei insbesondere das Benutzerassistenzsystem dazu eingerichtet ist, nach Ablauf der vorgesehenen Aufbereitungszeit auf der Projektionsfläche Informationen betreffend eine Position eines Medizinprodukts anzuzeigen, dessen vorgesehene Aufbereitungszeit abgelaufen ist.

Eine solche Anzeige ist beispielsweise bei der Aufbereitung von Endoskopen sinnvoll. Beispielsweise werden mehrere Endoskope in unterschiedlichen Spülkörben einer Aufbereitungsvorrichtung oder ggf. auch mehreren Aufbereitungsvorrichtungen aufbereitet. Jedem Endoskop ist dabei eine vorgesehene Aufbereitungszeit zugeordnet. Die Restdauer dieser vorgesehenen Aufbereitungszeit wird dann auf der Projektionsfläche angezeigt. Nach Ablauf der vorgesehenen Aufbereitungszeit zeigt die Datenbrille beispielsweise an, in welchem Spülkorb sich das zugehörige Endoskop befindet, oder die Position des Spülkorbs wird auf der Projektionsfläche der Datenbrille direkt im Blickfeld des Nutzers angezeigt.

Die Aufgabe wird außerdem gelöst durch ein Verfahren zum Überwachen der Aufbereitung von wiederverwendbaren Medizinprodukten, wobei eine Vielzahl von Typdatensätzen für verschiedene Typen von wiederverwendbaren Medizinprodukten und Informationen betreffend Anweisungen zur Wiederaufbereitung dieser Medizinprodukt-Typen auf einem Datenspeicher gespeichert sind und in jedem Typdatensatz Informationen betreffend ein Identifikationsmerkmal eines bestimmten Medizinprodukt-Typs vorhanden sind,
- wobei visuelle Informationen auf eine im Blickfeld eines Benutzers einer Datenbrille angeordnete Projektionsfläche projiziert werden,
- wobei mittels einer Kamera wenigstens ein Bild des wiederverwendbaren Medizinprodukts erfasst wird, die Bilddaten des Bilds mit den Informationen betreffend die Identifikationsmerkmale in den Typdatensätzen abgeglichen werden, ein Typ des wiederverwendbaren Medizinprodukts anhand eines in dem Bild vorhandenen Identifikationsmerkmals identifiziert wird und ein einem zugehörigen Typdatensatz zugeordnetes Typprofil aktiviert wird,
   wobei das Verfahren dadurch weitergebildet ist, dass
- ferner ein Nutzeridentifikationsmerkmal erfasst, mit auf dem Datenspeicher gespeicherten Nutzerdatensätzen, die jeweils Informationen betreffend wenigstens ein Nutzeridentifikationsmerkmal umfassen, abgeglichen und bei Übereinstimmung ein dem Nutzerdatensatz zugeordnetes Nutzerprofil aktiviert wird,
- wobei ferner ein Standortidentifikationsmerkmal erfasst, mit auf dem Datenspeicher gespeicherten Standortdatensätzen, die jeweils Informationen betreffend wenigstens ein Standortidentifikationsmerkmal umfassen, abgeglichen und bei Übereinstimmung ein dem Standortdatensatz zugeordnetes Standortprofil aktiviert wird,
- wobei der Datenspeicher Informationen betreffend Anweisungen zur Wiederaufbereitung des Medizinprodukts umfasst, die zumindest einem Typprofil, zumindest einem Nutzerprofil und zumindest einem Standortprofil zugeordnet sind, und wobei die dem aktiven Typprofil, dem aktiven Nutzerprofil und dem aktiven Standortprofil zugeordneten Anweisungen zur Wiederaufbereitung des Medizinprodukts als visuelle Informationen auf der Projektionsfläche angezeigt werden.

Auf das Verfahren zum Überwachen der Aufbereitung von wiederverwendbaren Medizinprodukten treffen gleiche o. ä. Vorteile zu wie sie bereits im Hinblick auf das Benutzerassistenzsystem erwähnt wurden, sodass auf Wiederholungen verzichtet werden soll.

Vorzugsweise ist das Verfahren dadurch weitergebildet, dass das Standortidentifikationsmerkmal in einem RFID- oder NFC-Transponder gespeichert ist, in einem Barcode oder einem DataMatrix-Code codiert ist und/oder aus einer äußeren Erscheinung zumindest eines ortsfesten Gegenstands besteht.

Vorzugsweise ist das Nutzeridentifikationsmerkmal ein Passwort und/oder ein biometrisches Merkmal des Nutzers.

Ferner ist vorzugsweise das Verfahren dadurch weitergebildet, dass Anweisungen betreffend die Wiederaufbereitung des Medizinprodukts als visuelle Informationen ausgegeben und durch Auswertung der von der Kamera aufgenommenen Bilddaten die Ausführung wenigstens einer dieser Anweisungen überwacht wird, wobei insbesondere bei fehlerhafter Ausführung der Anweisung eine Fehlermeldung ausgegeben wird.

Vorzugsweise ist das Verfahren ferner dadurch weitergebildet, dass mittels einer Lichtquelle Licht in einem Wellenlängenbereich emittiert wird, in dem medizinische Verunreinigungen, insbesondere Blut oder Proteine, zu Fluoreszenz angeregt werden, wobei mittels der Kamera Bilddaten in einem Wellenlängenbereich erfasst werden, in dem die Fluoreszenz der medizinischen Verunreinigungen auftritt, wobei ferner Fluoreszenzbilder erzeugt werden, insbesondere verstärkt werden, und diese Fluoreszenzbilder auf der Projektionsfläche angezeigt werden.

Gemäß einer Ausführungsform wird die Navigation in den visuellen Informationen, die auf der Projektionsfläche eingeblendet werden, durch Erkennung von Augenbewegungen, Gestenerkennung, Spracherkennung, und/oder mechanische Betätigung eines Eingabeelements am Gehäuse der Datenbrille gesteuert.

Vorzugsweise werden die laufenden und abgeschlossenen Wiederaufbereitungsvorgänge erfasst und dokumentiert, wobei die Informationen betreffend die laufenden und abgeschlossenen Wiederaufbereitungsvorgänge auf einem externen Datenspeicher gespeichert werden.

Gemäß einer weiteren vorteilhaften Ausführungsform ist das Verfahren dadurch weitergebildet, dass wenigstens einem Wiederaufbereitungsvorgang eine vorgesehene Aufbereitungszeit zugeordnet wird, wobei auf der Projektionsfläche die Restdauer der vorgesehenen Aufbereitungszeit angezeigt wird, wobei insbesondere bei Ablauf der vorgesehenen Aufbereitungszeit auf der Projektionsfläche Informationen betreffend eine Position eines Medizinprodukts angezeigt werden, dessen vorgesehene Aufbereitungszeit abgelaufen ist.

Die Aufgabe wird ferner gelöst durch ein Computerprogramm mit Programmcode-Mitteln, die angepasst sind, ein Verfahren mit einem oder mehreren der oben genannten Merkmale auszuführen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen jeweils in schematischer und vereinfachter Darstellung:
- Fig. 1: ein Benutzerassistenzsystem,
- Fig. 2: eine Datenbrille,
- Fig. 3: eine Struktur eines Datenspeichers,
- Fig. 4: eine Struktur eines Typdatensatzes,
- Fig. 5: eine Struktur eines Nutzerdatensatzes,
- Fig. 6: eine Struktur des Standortdatensatzes,
- Fig. 7: eine Identifikation eines Typs von wiederverwendbaren Medizinprodukten,
- Fig. 8: eine Identifikation eines Nutzers,
- Fig. 9: eine Identifikation eines Standorts,
- Fig. 10: eine Fluoreszenzfunktion der Datenbrille.

In den Zeichnungen sind jeweils gleiche oder gleichartige Elemente und/oder Teile mit denselben Bezugsziffern versehen, so dass von einer erneuten Vorstellung jeweils abgesehen wird.

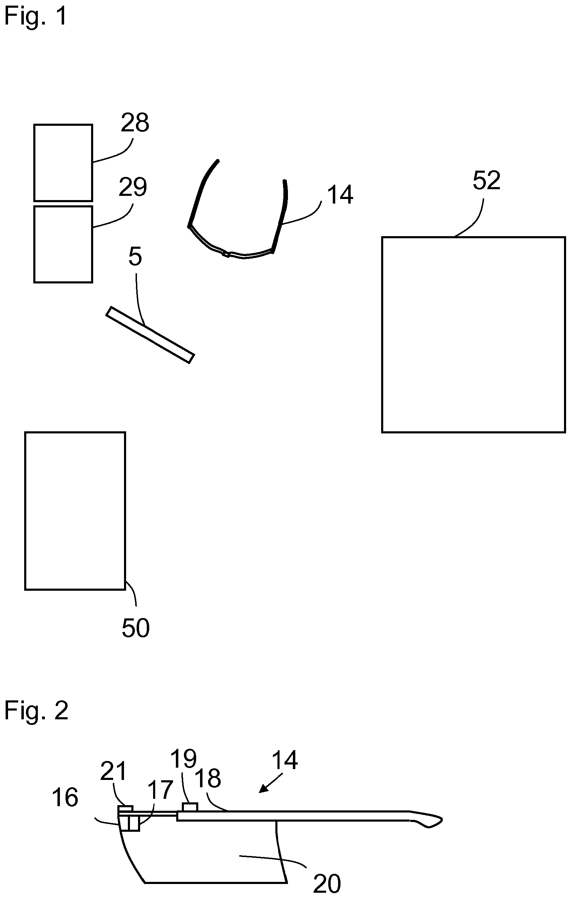

Fig. 1 zeigt schematisch und vereinfacht ein Benutzerassistenzsystem umfassend eine Datenbrille 14 und einen Datenspeicher 28. Der Datenspeicher 28 ist in diesem Beispiel getrennt von der Datenbrille 14, beispielsweise als Teil eines Servers, welcher mittels einer drahtlosen Datenverbindung mit der Datenbrille 14 verbunden ist, dargestellt. Das Benutzerassistenzsystem wird zur Aufbereitung eines wiederverwendbaren Medizinprodukts 5 verwendet, wobei die Aufbereitung beispielsweise an mehreren Standorten 50, 52 durchgeführt wird. Bei den Standorten 50, 52 handelt es sich beispielsweise um eine Aufbereitungsmaschine und ein Spülbecken.

Auf dem externen Datenspeicher 29 sind Informationen betreffend die laufenden und abgeschlossenen Wiederaufbereitungsvorgänge gespeichert, so dass die Aufbereitungen dokumentiert werden.

In Fig. 2 ist die Datenbrille 14 schematisch und vereinfacht in einer Seitenansicht dargestellt. Die Datenbrille 14 umfasst ein Gehäuse 18 und eine Projektionsfläche 20, auf der die Datenbrille 14 visuelle Informationen in Form von Texten, Bildern, Videos oder ähnlichem einblenden kann. Die Projektionsfläche 20 ist im Bereich des sichtbaren Lichts transparent, so dass das Sichtfeld des Anwenders im Wesentlichen nicht eingeschränkt wird.

Die Datenbrille 14 umfasst zudem eine in Blickrichtung des Anwenders gerichtete Kamera 16, eine im Wesentlichen den Augenbereich des Anwenders erfassende Kamera 17, ein oder mehrere Mikrofone 19 und eine Lichtquelle 21. Zusätzlich umfasst die Datenbrille 14 eine im Gehäuse 18 angeordnete Datenverarbeitungsvorrichtung, auf der ein Computerprogramm zur Steuerung der Funktionen der Datenbrille 14 gespeichert und ausführbar ist. Mittels der Kamera 16 erfasst die Datenbrille 14 Bilddaten und wertet diese anschließend in der Datenverarbeitungsvorrichtung aus, um so bestimmte Identifikationsmerkmale 6, 41, 51, 53, Gegenstände oder Personen zu erkennen. Zusätzlich kann auch die Position von Objekten im Raum bestimmt werden. Dies ermöglicht es, auf der Projektionsfläche 20 Bildsignale einzublenden, durch die diese Objekte aus der Sicht des Anwenders hervorgehoben werden.

Die genaue Position der Kameras 16 und 17, des Mikrofons oder der Mikrofone 19 und der Lichtquelle 21 auf oder in dem Gehäuse 18 kann abweichend von der Darstellung in Fig. 2 gewählt werden, solange ihre Funktion hierdurch nicht eingeschränkt wird. Auch die Form der Projektionsfläche 20 kann von der in Fig. 2 gezeigten Form abweichen. In dem Gehäuse 18 befindet sich zudem ein Empfänger, mit dem ein RFID- oder NFC-Signal empfangen werden kann.

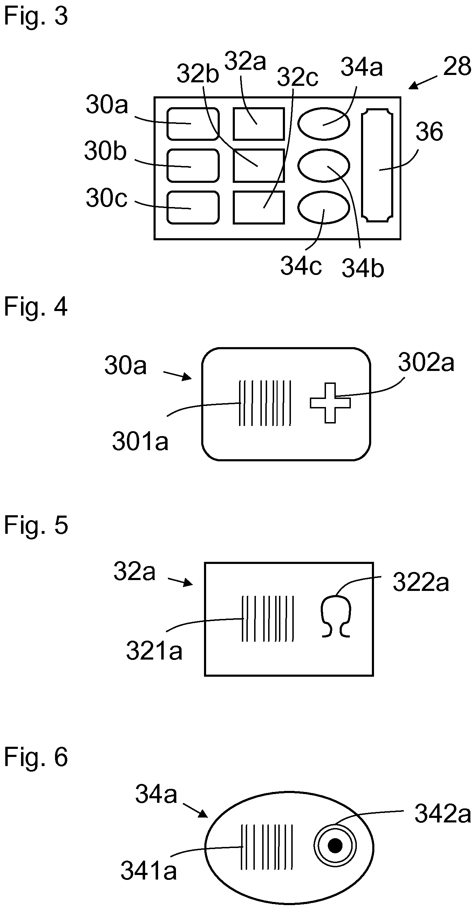

In Fig. 3 ist die Struktur eines Datenspeichers 28, wie er für das Benutzerassistenzsystem eingesetzt werden kann, schematisch dargestellt. Auf dem Datenspeicher 28 sind mehrere Typdatensätze 30a, 30b, 30c, mehrere Nutzerdatensätze 32a, 32b, 32c, mehrere Standortdatensätze 34a, 34b, 34c und Informationen 36 betreffend Anweisungen zur Aufbereitung gespeichert. Jeder Typdatensatz 30a, 30b, 30c umfasst Informationen betreffend einen spezifischen Typ von wiederverwendbarem Medizinprodukt 5, jeder Nutzerdatensatz 32a, 32b, 32c Informationen betreffend einen spezifischen Nutzer und jeder Standortdatensatz 34a, 34b, 34c Informationen betreffend einen spezifischen Standort.

In den Fig. 4, 5 und 6 sind der Typdatensatz 30a, der Nutzerdatensatz 32a und der Standortdatensatz 34a schematisch und beispielhaft dargestellt. Jeder Datensatz 30a, 32a, 34a umfasst dabei jeweils Informationen 301a, 321a, 341a betreffend wenigstens ein Identifikationsmerkmal 6, 41, 51, 53. Dabei können in einem Datensatz 30a, 32a, 34a auch Informationen 301a, 321a, 341a betreffend mehrere Identifikationsmerkmale gespeichert sein. Beispielsweise sind im Datensatz 30a Informationen 301a über ein erstes Identifikationsmerkmal 6, welches in einem Barcode codiert ist, und ein zweites Identifikationsmerkmal, welches in einem DataMatrix-Code codiert ist, gespeichert, wobei beide dem gleichen Typ von Medizinprodukt 5 zugeordnet sind. Zusätzlich umfassen die Datensätze 30a, 32a, 34a auch das Typprofil 302a, das Nutzerprofil 322a und das Standortprofil 342a. Die in den Fig. 3, 4, 5 und 6 gezeigte Struktur des Datenspeichers 28 ist lediglich beispielhaft. So könnten beispielsweise die Informationen 36 auch in den Typdatensätzen 30a, 30b, 30c gespeichert sein.

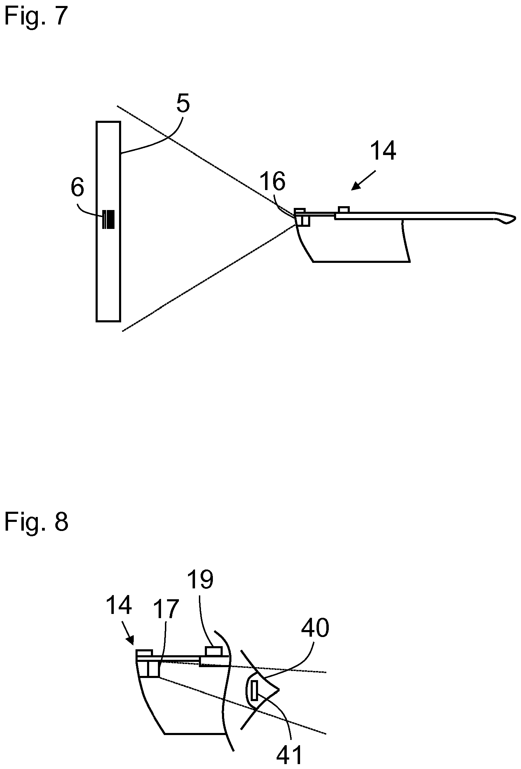

In Fig. 7 ist schematisch und vereinfacht dargestellt, wie die Erfassung eines Identifikationsmerkmals 6 eines wiederverwendbaren Medizinprodukts 5 funktioniert. In diesem Beispiel ist das Identifikationsmerkmal 6 in einem Barcode codiert, der mittels der Kamera 16 erfasst wird. Anschließend wird dieser Barcode mit den in den Typdatensätzen 30a, 30b, 30c gespeicherten Informationen 301a betreffend die Identifikationsmerkmale 6 abgeglichen. Stimmt das erfasste Identifikationsmerkmal 6 mit dem in den Informationen 301a gespeichertem Identifikationsmerkmal 6 überein, so wird das Typprofil 302a aktiviert, das demselben Typdatensatz 30a wie die Informationen 301a zugeordnet ist. Auf der Projektionsfläche 20 der Datenbrille 14 werden nun visuelle Informationen eingeblendet, die die Aufbereitung des zugehörigen Typs von wiederverwendbarem Medizinprodukt 5 betreffen.

Im Zusammenhang mit Fig. 8 soll die Identifikation eines Nutzers über ein Nutzeridentifikationsmerkmal 41 beschrieben werden. In dem in Fig. 7 gezeigten Beispiel handelt es sich bei dem Nutzeridentifikationsmerkmal 41 um ein Irismuster des Auges 40 eines Nutzers. Mittels der Kamera 17 wird ein Bild der Iris erfasst und die Bilddaten werden mit den in den Nutzerdatensätzen 32a, 32b, 32c gespeicherten Information 321a verglichen. Bei Übereinstimmung wird ein entsprechendes Nutzerprofil 322a aktiviert und die auf der Projektionsfläche 20 angezeigten Informationen werden so ausgewählt oder eingeschränkt, dass nur die für den erkannten Nutzer relevanten Informationen eingeblendet werden.

Beispielsweise kann zur Nutzeridentifikation auch eine Sprach- und Stimmerkennung verwendet werden. Dabei wird der Anwender aufgefordert, ein Passwort zu sprechen. Mittels des Mikrophons 19 wird das gesprochene Passwort des Nutzers erfasst, und bevorzugt sowohl das Passwort selber als auch die Stimme des Nutzers mit den Information 321a verglichen. Stimmen beide Merkmale, also Passwort und Stimme, überein, wird das Nutzerprofil 321a aktiviert.

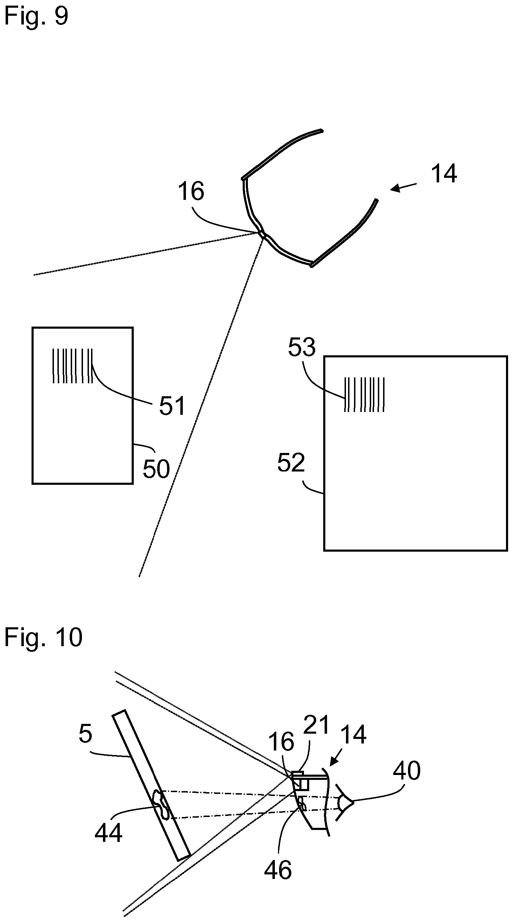

In Fig. 9 ist die Identifikation eines Standortidentifikationsmerkmals 51, 53 schematisch dargestellt. Es sind zwei Standorte 50, 52 gezeigt, bei denen es sich beispielsweise um eine Aufbereitungsmaschine und ein Spülbecken handeln kann. Jeder Standort 50, 52 weist ein Standortidentifikationsmerkmal 51, 53 auf. Wird ein Standortidentifikationsmerkmal 51 von der Datenbrille 14 erfasst, so wird das Standortidentifikationsmerkmal 51 mit den auf den Standortdatensätzen 34a, 34b, 34c gespeicherten Informationen 341a verglichen und bei Übereinstimmung ein zugehöriges Standortprofil 342a aktiviert. Daraufhin werden dem Nutzer nur diejenigen Informationen eingeblendet, die für die Aufbereitung des Medizinprodukts 5 an dem derzeitigen Standort 50 relevant sind. Wechselt der Nutzer den Standort, so dass er sich nun bei Standort 52 aufhält, wird das Standortidentifikationsmerkmal 53 erfasst und das entsprechende Standortprofil, das diesem neuen Standort 52 entspricht, aktiviert.

Somit werden dem jeweiligen Nutzer an seinem derzeitigen Standort 50, 52 nur diejenigen Informationen eingeblendet, die für diesen Nutzer an diesem Standort 50, 52 zur Aufbereitung dieses Typs von Medizinprodukt 5 von Bedeutung sind. Hierdurch wird eine Optimierung des Ablaufs der Aufbereitung und eine Reduktion der Fehler bei der Aufbereitung erreicht.

Fig. 10 zeigt, wie mittels der Kamera das Ergebnis der Aufbereitung kontrolliert werden kann. Mittels der Lichtquelle 21 wird Licht in einem Wellenbereich emittiert, bei dem Verunreinigungen 44, insbesondere Blut- oder Proteinreste, zu Fluoreszenz angeregt werden. Der beleuchtete Bereich ist in Fig. 10 durch gepunktete Linien dargestellt. Durch die Kamera 16 wird ein Fluoreszenzsignal der Verunreinigungen 44 erfasst und anschließend in der Datenverarbeitungsvorrichtung der Datenbrille 14 verstärkt. Die Datenbrille 14 zeigt dann ein verstärktes Fluoreszenzbild 46 auf der Projektionsfläche 20 an. Der Anwender, dargestellt durch ein Auge 40, nimmt also eine verstärkte Fluoreszenz der Blut- oder Proteinreste wahr. Auf diese Weise kann der Anwender Blutspuren oder Restproteine auf einem zu reinigenden wiederverwendbaren Medizinprodukt 5 leicht erkennen und so den Erfolg einer Aufbereitung überprüfen.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 5: Medizinprodukt
- 6: Identifikationsmerkmal
- 14: Datenbrille
- 16: Kamera
- 17: Kamera
- 18: Gehäuse
- 19: Mikrophon
- 20: Projektionsfläche
- 21: Lichtquelle
- 28: Datenspeicher
- 29: Datenspeicher
- 30a, 30b, 30c: Typdatensatz
- 32a, 32b, 32c: Nutzerdatensatz
- 34a, 34b, 34c: Standortdatensatz
- 36: Informationen
- 40: Auge
- 41: Nutzeridentifikationsmerkmal
- 44: Verunreinigungen
- 46: Fluoreszenzbilder
- 50: erster Standort
- 51: erstes Standortidentifikationsmerkmal
- 52: zweiter Standort
- 53: zweites Standortidentifikationsmerkmal
- 301a: Informationen
- 302a: Typprofil
- 321a: Informationen
- 322a: Nutzerprofil
- 341a: Informationen
- 342a: Standortprofil

## Patentansprüche

1. Benutzerassistenzsystem für wiederverwendbare Medizinprodukte (5), umfassend eine Datenbrille (14) mit einer Kamera (16), einem bildgebenden optischen Modul mit einer Projektionsfläche (20), wobei das Benutzerassistenzsystem ferner einen Datenspeicher (28) umfasst, auf dem eine Vielzahl von Typdatensätzen (30a, 30b, 30c), jeweils umfassend Informationen (301a) betreffend ein Identifikationsmerkmal (6) eines bestimmten Medizinprodukt-Typs, und Informationen (36) betreffend Anweisungen zur Wiederaufbereitung dieser Medizinprodukt-Typen gespeichert sind, wobei
- das bildgebende optische Modul dazu eingerichtet ist, visuelle Informationen auf die im Blickfeld eines Nutzers der Datenbrille (14) angeordnete Projektionsfläche (20) zu projizieren,
- das Benutzerassistenzsystem dazu eingerichtet ist, ein Bild eines wiederverwendbaren Medizinprodukts (5) mit der Kamera (16) zu erfassen, die Bilddaten des Bilds mit den Informationen (301a) betreffend die Identifikationsmerkmale (6) in den Typdatensätzen (30a, 32a, 34a) abzugleichen, einen Typ des wiederverwendbaren Medizinprodukts (5) anhand eines in dem Bild vorhandenen Identifikationsmerkmals (6) zu identifizieren und ein einem zugehörigen Typdatensatz (30a) zugeordnetes Typprofil (302a) zu aktivieren,
wobei das Benutzerassistenzsystem ferner dazu eingerichtet ist,
- ein Nutzeridentifikationsmerkmal (41) zu erfassen und mit auf dem Datenspeicher (28) gespeicherten Nutzerdatensätzen (32a, 32b, 32c), die jeweils Informationen (321a) betreffend wenigstens ein Nutzeridentifikationsmerkmal (41) umfassen, abzugleichen und bei Übereinstimmung ein dem Nutzerdatensatz (32a, 32b, 32c) zugeordnetes Nutzerprofil (322a) zu aktivieren,
- ein Standortidentifikationsmerkmal (51, 53) zu erfassen und mit auf dem Datenspeicher (28) gespeicherten Standortdatensätzen (34a, 34b, 34c), die jeweils Informationen (341a) betreffend wenigstens ein Standortidentifikationsmerkmal (51, 53) umfassen, abzugleichen und bei Übereinstimmung ein dem Standortdatensatz (34a, 34b, 34c) zugeordnetes Standortprofil (342a) zu aktivieren,
- **dadurch gekennzeichnet, dass**
der Datenspeicher (28) Informationen (36) betreffend Anweisungen zur Wiederaufbereitung von Medizinprodukten (5) umfasst, die zumindest einem Typprofil (302a), zumindest einem Nutzerprofil (322a) und zumindest einem Standortprofil (342a) zugeordnet sind, und wobei die dem aktiven Typprofil (302a), dem aktiven Nutzerprofil (322a) und dem aktiven Standortprofil (342a) zugeordneten Anweisungen zur Wiederaufbereitung des Medizinprodukts (5) als visuelle Informationen auf der Projektionsfläche (20) angezeigt werden.

2. Benutzerassistenzsystem nach Anspruch 1, **dadurch gekennzeichnet, dass** das Standortidentifikationsmerkmal (51, 53)
- in einem RFID- oder NFC-Transponder gespeichert ist,
- in einem Barcode oder einem DataMatrix-Code codiert ist und/oder
- aus einer äußeren Erscheinung zumindest eines ortsfesten Gegenstands besteht.

3. Benutzerassistenzsystem nach Anspruch 1 oder 2, **dadurch gekennzeichnet, dass** das Nutzeridentifikationsmerkmal (41) ein Passwort und/oder ein biometrisches Merkmal des Nutzers ist.

4. Benutzerassistenzsystem nach einem der Ansprüche 1 bis 3, **dadurch gekennzeichnet, dass** das Benutzerassistenzsystem dazu eingerichtet ist, Anweisungen betreffend die Wiederaufbereitung des Medizinprodukts als visuelle Informationen auszugeben und durch Auswertung der von der Kamera (16) aufgenommenen Bilddaten eine Ausführung dieser Anweisung zu überwachen, wobei insbesondere bei fehlerhafter Ausführung der Anweisung das Benutzerassistenzsystem zur Ausgabe einer Fehlermeldung eingerichtet ist.

5. Benutzerassistenzsystem nach einem der Ansprüche 1 bis 4, **dadurch gekennzeichnet, dass** eine Lichtquelle (21) umfasst ist, welche in einem Wellenlängenbereich emittiert, in dem medizinische Verunreinigungen (44), insbesondere Blut oder Proteine, zu Fluoreszenz angeregt werden, wobei die Kamera (16) zur Bilderfassung in einem Wellenlängenbereich eingerichtet ist, in dem die Fluoreszenz der medizinischen Verunreinigungen (44) auftritt, wobei das Benutzerassistenzsystem ferner zur Erzeugung von Fluoreszenzbildern (46), insbesondere zur Verstärkung von Fluoreszenzbildern (46), und zur Darstellung derselben auf der Projektionsfläche (20) eingerichtet ist.

6. Benutzerassistenzsystem nach einem der Ansprüche 1 bis 5, **dadurch gekennzeichnet, dass** die Navigation in den visuellen Informationen, die auf der Projektionsfläche (20) eingeblendet werden, durch
- Erkennung von Augenbewegungen,
- Gestenerkennung,
- Spracherkennung und/oder
- mechanische Betätigung eines Eingabeelements am Gehäuse (18) der Datenbrille (14)
steuerbar ist.

7. Benutzerassistenzsystem nach einem der Ansprüche 1 bis 6, **dadurch gekennzeichnet, dass** das Benutzerassistenzsystem zur Erfassung und Dokumentation der laufenden und abgeschlossenen Wiederaufbereitungsvorgänge eingerichtet ist, wobei das Benutzerassistenzsystem ferner einen externen Datenspeicher (29) umfasst, der zur Speicherung der Informationen betreffend die laufenden und abgeschlossenen Wiederaufbereitungsvorgänge eingerichtet ist.

8. Benutzerassistenzsystem nach einem der Ansprüche 1 bis 7, **dadurch gekennzeichnet, dass** das Benutzerassistenzsystem dazu eingerichtet ist, wenigstens einem Wiederaufbereitungsvorgang eine vorgesehene Aufbereitungszeit zuzuordnen, und auf der Projektionsfläche (20) eine Restdauer der vorgesehenen Aufbereitungszeit anzuzeigen, wobei insbesondere das Benutzerassistenzsystem dazu eingerichtet ist, nach Ablauf der vorgesehenen Aufbereitungszeit auf der Projektionsfläche (20) Informationen betreffend eine Position eines Medizinprodukts (5) anzuzeigen, dessen vorgesehene Aufbereitungszeit abgelaufen ist.

9. Verfahren zum Überwachen der Aufbereitung von wiederverwendbaren Medizinprodukten (5), wobei eine Vielzahl von Typdatensätzen (30a, 30b, 30c) für verschiedene Typen von wiederverwendbaren Medizinprodukten (5) und Informationen (36) betreffend Anweisungen zur Wiederaufbereitung dieser Medizinprodukt-Typen auf einem Datenspeicher (28) gespeichert sind und in jedem Typdatensatz (30a, 30b, 30c) Informationen (301a) betreffend ein Identifikationsmerkmal (6) eines bestimmten Medizinprodukt-Typs vorhanden sind,
- wobei visuelle Informationen auf eine im Blickfeld eines Benutzers einer Datenbrille (14) angeordnete Projektionsfläche (20) projiziert werden,
- wobei mittels einer Kamera (16) wenigstens ein Bild des wiederverwendbaren Medizinprodukts (5) erfasst wird, die Bilddaten des Bilds mit den Informationen (301a) betreffend die Identifikationsmerkmale (6) in den Typdatensätzen (30a, 30b, 30c) abgeglichen werden, ein Typ des wiederverwendbaren Medizinprodukts (5) anhand eines in dem Bild vorhandenen Identifikationsmerkmals (6) identifiziert wird und ein einem zugehörigen Typdatensatz (30a, 30b, 30c) zugeordnetes Typprofil (302a) aktiviert wird,
wobei
- ferner ein Nutzeridentifikationsmerkmal (41) erfasst, mit auf dem Datenspeicher (28) gespeicherten Nutzerdatensätzen (32a, 32b, 32c), die jeweils Informationen (321a) betreffend wenigstens ein Nutzeridentifikationsmerkmal (41) umfassen, abgeglichen und bei Übereinstimmung ein dem Nutzerdatensatz (32a, 32b, 32c) zugeordnetes Nutzerprofil (322a) aktiviert wird,
- wobei ferner ein Standortidentifikationsmerkmal (51, 53) erfasst, mit auf dem Datenspeicher (28) gespeicherten Standortdatensätzen (34a, 34b, 34c), die jeweils Informationen (341a) betreffend wenigstens ein Standortidentifikationsmerkmal (51, 53) umfassen, abgeglichen und bei Übereinstimmung ein dem Standortdatensatz (34a, 34b, 34c) zugeordnetes Standortprofil (342a) aktiviert wird,
- **dadurch gekennzeichnet, dass**
der Datenspeicher (28) Informationen (36) betreffend Anweisungen zur Wiederaufbereitung des Medizinprodukts (5) umfasst, die zumindest einem Typprofil (302a), zumindest einem Nutzerprofil (322a) und zumindest einem Standortprofil (342a) zugeordnet sind, und wobei die dem aktiven Typprofil (302a), dem aktiven Nutzerprofil (322a) und dem aktiven Standortprofil (342a) zugeordneten Anweisungen zur Wiederaufbereitung des Medizinprodukts (5) als visuelle Informationen auf der Projektionsfläche (20) angezeigt werden.

10. Verfahren nach Anspruch 9, **dadurch gekennzeichnet, dass** das Standortidentifikationsmerkmal (51, 53)
- in einem RFID- oder NFC-Transponder gespeichert ist,
- in einem Barcode oder einem DataMatrix-Code codiert ist und/oder
- aus einer äußeren Erscheinung zumindest eines ortsfesten Gegenstands besteht.

11. Verfahren nach Anspruch 9 oder 10, **dadurch gekennzeichnet, dass** das Nutzeridentifikationsmerkmal (41) ein Passwort und/oder ein biometrisches Merkmal des Nutzers ist.

12. Verfahren nach einem der Ansprüche 9 bis 11, **dadurch gekennzeichnet, dass** Anweisungen betreffend die Wiederaufbereitung des Medizinprodukts (5) als visuelle Informationen ausgegeben und durch Auswertung der von der Kamera (16) aufgenommenen Bilddaten die Ausführung wenigstens einer dieser Anweisungen überwacht wird, wobei insbesondere bei fehlerhafter Ausführung der Anweisung eine Fehlermeldung ausgegeben wird.

13. Verfahren nach einem der Ansprüche 9 bis 12, **dadurch gekennzeichnet, dass** mittels einer Lichtquelle (21) Licht in einem Wellenlängenbereich emittiert wird, in dem medizinische Verunreinigungen (44), insbesondere Blut oder Proteine, zu Fluoreszenz angeregt werden, wobei mittels der Kamera (16) Bilddaten in einem Wellenlängenbereich erfasst werden, in dem die Fluoreszenz der medizinischen Verunreinigungen (44) auftritt, wobei ferner Fluoreszenzbildern (46) erzeugt werden, insbesondere verstärkt werden, und diese Fluoreszenzbilder (46) auf der Projektionsfläche (20) angezeigt werden.

14. Verfahren nach einem der Ansprüche 9 bis 13, **dadurch gekennzeichnet, dass** die Navigation in den visuellen Informationen, die auf der Projektionsfläche (20) eingeblendet werden, durch
- Erkennung von Augenbewegungen,
- Gestenerkennung,
- Spracherkennung, und/oder
- mechanische Betätigung eines Eingabeelements am Gehäuse (18) der Datenbrille (14)
gesteuert wird.

15. Verfahren nach einem der Ansprüche 9 bis 14, **dadurch gekennzeichnet, dass** die laufenden und abgeschlossenen Wiederaufbereitungsvorgänge erfasst und dokumentiert werden, wobei die Informationen betreffend die laufenden und abgeschlossenen Wiederaufbereitungsvorgänge auf einem externen Datenspeicher (29) gespeichert werden.

16. Verfahren nach einem der Ansprüche 9 bis 15, **dadurch gekennzeichnet, dass** wenigstens einem Wiederaufbereitungsvorgang eine vorgesehene Aufbereitungszeit zugeordnet wird, wobei auf der Projektionsfläche (20) die Restdauer der vorgesehenen Aufbereitungszeit angezeigt wird, wobei insbesondere bei Ablauf der vorgesehenen Aufbereitungszeit auf der Projektionsfläche (20) Informationen betreffend eine Position eines Medizinprodukts (5) angezeigt werden, dessen vorgesehene Aufbereitungszeit abgelaufen ist.

17. Computerprogramm mit Programmcode-Mitteln, die bewirken, dass die Vorrichtung nach einem der Ansprüche 1 bis 8 die Verfahrensschritte nach einem der Ansprüche 9 bis 16 ausführt.

## Claims

1. A user assistance system for reusable medical devices (5) comprising data glasses (14) with a camera (16), an imaging optical module with a projection surface (20), wherein the user assistance system furthermore comprises a data memory (28) in which a plurality of type datasets (30a, 30b, 30c) are saved that each comprise information (301a) relating to an identifying feature (6) of a specific medical device type, and information (36) relating to instructions for reprocessing these medical device types, wherein:
- the imaging optical module is configured to project visual information onto the projection surface (20) arranged within the field of vision of a user of the data glasses (14),
- the user assistance system is configured to record an image of a reusable medical device (5) with the camera (16), to compare the image data of the image with the information (301a) relating to the identifying features (6) in the type datasets (30a, 32a, 34a), to identify a type of the reusable medical device (5) using an identifying feature (6) available within the image, and to activate a type profile (302a) assigned to an associated type dataset (30a),
wherein the user assistance system is furthermore configured to:
- record a user identifying feature (41) and compare it with user datasets (32a, 32b, 32c) saved on the data memory (28) which each comprise information (321a) relating to at least one user identifying feature (41) and, in the event of correspondence, to activate a user profile (322a) assigned to the user dataset (32a, 32b, 32c),
- record a location identifying feature (51, 53) and compare it with location datasets (34a, 34b, 34c) saved on the data memory (28) which each comprise information (341a) relating to at least one location identifying feature (51, 53) and, in the event of correspondence, to activate a location profile (342a) assigned to the location dataset (34a, 34b, 34c),
- **characterized in that** the data memory (28) comprises information (36) relating to instructions for reprocessing medical devices (5) that are assigned to at least one type profile (302a), at least one user profile (322a) and at least one location profile (342a), and wherein the instructions for reprocessing the medical device (5) assigned to the active type profile (302a), the active user profile (322a) and the active location profile (342a) are displayed as visual information on the projection surface (20).

2. The user assistance system according to claim 1, **characterized in that** the location identifying feature (51, 53):
- is saved in an RFID or NFC transponder,
- is encoded in a barcode or a data matrix code, and/or
- consists of an external appearance of at least one stationary object.

3. The user assistance system according to claim 1 and 2, **characterized in that** the user identifying feature (41) is a password and/or a biometric feature of the user.

4. The user assistance system according to one of claims 1 to 3, **characterized in that** the user assistance system is configured to output instructions relating to the reprocessing of the medical device as visual information and to monitor execution of these instructions by evaluating the image data recorded by the camera (16), wherein in particular the user assistance system is configured to output an error message in the event that the instruction is incorrectly executed.

5. The user assistance system according to one of claims 1 to 4, **characterized in that** a light source (21) is comprised that emits within a wavelength range in which medical contaminants (44), in particular blood or proteins, are excited to fluoresce, wherein the camera (16) is configured to record images within a wavelength range in which the fluorescence of the medical contaminants (44) occurs, wherein the user assistance system is furthermore configured to generate fluorescent images (46), in particular to amplify fluorescent images (46), and to show them on the projection surface (20).

6. The user assistance system according to one of claims 1 to 5, **characterized in that** the navigation within the visual information that appears on the projection surface (20) can be controlled by:
- recognizing eye movements,
- gesture recognition,
- speech recognition and/or
- mechanically actuating an input element on the housing (18) of the data glasses (14).

7. The user assistance system according to one of claims 1 to 6, **characterized in that** the user assistance system is configured to record and document the ongoing and completed reprocessing procedures, wherein the user assistance system furthermore comprises an external data memory (29) that is configured to save the information relating to the ongoing and completed reprocessing procedures.

8. The user assistance system according to one of claims 1 to 7, **characterized in that** the user assistance system is configured to assign a designated processing time to at least one reprocessing procedure and to display a remaining duration of the designated processing time on the projection surface (20), wherein in particular upon the expiration of the designated processing time, the user assistance system is configured to display information on the projection surface (20) relating to a position of a medical device (5) whose designated processing time has expired.

9. A method for monitoring the processing of reusable medical devices (5), wherein a plurality of type datasets (30a, 30b, 30c) for different types of reusable medical devices (5) and information (36) relating to instructions on the reprocessing of these medical device types are saved in a data memory (28), and information (301a) is present in each type dataset (30a, 30b, 30c) relating to an identifying feature (6) of a specific medical device type,
- wherein visual information is projected onto a projection surface (20) arranged in the field of vision of a user of data glasses (14),
- wherein at least one image of the reusable medical device (5) is recorded with a camera (16), the image data of the image are compared with the information (301a) relating to the identifying features (6) in the type datasets (30a, 30b, 30c), a type of the reusable medical device (5) is identified using an identifying feature (6) available within the image, and a type profile (302a) assigned to an associated type dataset (30a, 30b, 30c) is activated,
wherein:
- furthermore, a user identifying feature (41) is recorded and compared with user datasets (32a, 32b, 32c) saved on the data memory (28) which each comprise information (321a) relating to at least one user identifying feature (41) and, in the event of correspondence, a user profile (322a) assigned to the user dataset (32a, 32b, 32c) is activated,
- wherein furthermore, a location identifying feature (51, 53) is recorded and compared with location datasets (34a, 34b, 34c) saved on the data memory (28) which each comprise information (341a) relating to at least one location identifying feature (51, 53) and, in the event of correspondence, a location profile (342a) assigned to the location dataset (34a, 34b, 34c) is activated,
- **characterized in that** the data memory (28) comprises information (36) relating to instructions for reprocessing the medical device (5) that is assigned to at least one type profile (302a), at least one user profile (322a) and at least one location profile (342a), and wherein the instructions for reprocessing the medical device (5) assigned to the active type profile (302a), the active user profile (322a) and the active location profile (342a) are displayed as visual information on the projection surface (20).

10. The method according to claim 9, **characterized in that** the location identifying feature (51, 53):
- is saved in an RFID or NFC transponder,
- is encoded in a barcode or a data matrix code and/or
- consists of an external appearance of at least one stationary object.

11. The method according to claim 9 or 10, **characterized in that** the user identifying feature (41) is a password and/or a biometric feature of the user.

12. The method according to one of claims 9 to 11, **characterized in that** instructions relating to the reprocessing of the medical device (5) are output as visual information, and the execution of at least one of these instructions is monitored by evaluating the image data recorded by the camera (16), wherein in particular an error message is output in the event that the instruction is incorrectly executed.

13. The method according to one of claims 9 to 12, **characterized in that** light is emitted by means of a light source (21) within a wavelength range in which medical contaminants (44), in particular blood or proteins, are excited to fluoresce, wherein by means of the camera (16), image data are recorded within a wavelength range in which the fluorescence of the medical contaminants (44) occurs, wherein furthermore fluorescent images (46) are generated, in particular amplified, and these fluorescent images (46) are displayed on the projection surface (20).

14. The method according to one of claims 9 to 13, **characterized in that** the navigation within the visual information that appears on the projection surface (20) is controlled by:
- recognizing eye movements,
- gesture recognition,
- speech recognition, and/or
- mechanically actuating an input element on the housing (18) of the data glasses (14).

15. The method according to one of claims 9 to 14, **characterized in that** the ongoing and completed reprocessing procedures are recorded and documented, wherein the information relating to the ongoing and completed reprocessing procedures are saved on an external data memory (29).

16. The method according to one of claims 9 to 15, **characterized in that** a designated processing time is assigned to at least one reprocessing procedure, wherein the remaining duration of the designated processing time is displayed on the projection surface (20), wherein in particular upon the expiration of the designated processing time, information is displayed on the projection surface (20) relating to a position of a medical device (5) whose designated processing time has expired.

17. A computer program with program code means, which cause the means according to one of claims 1 to 8 to execute the method steps according to one of claims 9 to 16.

## Revendications

1. Système d'assistance à un utilisateur pour des produits médicaux (5) réutilisables, comprenant des lunettes (14) à données ayant une caméra (16), un module optique d'imagerie ayant une surface de projection (20), le système d'assistance à un utilisateur comprenant en outre une mémoire de données (28) sur laquelle sont mémorisés une pluralité d'enregistrements (30a, 30b, 30c) de données de type, comprenant chacun des informations (301a) concernant une caractéristique d'identification (6) d'un type de produit médical déterminé, et des informations (36) concernant des instructions pour le retraitement de ces types de produits médicaux, dans lequel
- le module optique d'imagerie est conçu pour projeter des informations visuelles sur la surface de projection (20) disposée dans le champ de vision d'un utilisateur des lunettes (14) à données,
- le système d'assistance à un utilisateur est conçu pour saisir une image d'un produit médical réutilisable (5) avec la caméra (16), pour comparer les données d'image de l'image avec les informations (301a) concernant les caractéristiques d'identification (6) dans les enregistrements (30a, 32a, 34a) de données de type, pour identifier un type du produit médical (5) réutilisable à l'aide d'une caractéristique d'identification (6) présente dans l'image et pour activer un profil de type (302a) associé à un enregistrement (30a) de données de type correspondant,
le système d'assistance à un utilisateur étant en outre conçu pour
- détecter une caractéristique (41) d'identification d'utilisateur et la comparer avec des enregistrements (32a, 32b, 32c) de données d'utilisateur enregistrés sur la mémoire de données (28), qui comprennent chacun des informations (321a) concernant au moins une caractéristique (41) d'identification d'utilisateur, et en cas de concordance, activer un profil d'utilisateur (322a) associé à l'enregistrement (32a, 32b, 32c) de données d'utilisateur,
- détecter une caractéristique (51, 53) d'identification de localisation et la comparer avec des ensembles (34a, 34b, 34c) de données de localisation enregistrés sur la mémoire de données (28), qui comprennent chacun des informations (341a) concernant au moins une caractéristique (51, 53) d'identification de localisation, et en cas de concordance, activer un profil de localisation (342a) associé à l'ensemble de données de localisation (34a, 34b, 34c),
- **caractérisé en ce que**
la mémoire de données (238) comprend des informations (36) concernant des instructions de retraitement de produits médicaux (5) qui sont associées à au moins un profil de type (302a), au moins un profil utilisateur (322a) et au moins un profil de localisation (342a), et dans lequel les instructions de retraitement du produit médical (5) associées au profil de type actif (302a), au profil utilisateur actif (322a) et au profil de localisation actif (342a) sont affichées sous forme d'informations visuelles sur la surface de projection (20).

2. Système d'assistance à un utilisateur selon la revendication 1, **caractérisé en ce que** la caractéristique (51, 53) d'identification de localisation
- est stockée dans un transpondeur RFID ou NFC,
- est codée dans un code à barres ou un code DataMatrix et/ou
- consiste en une apparence extérieure d'au moins un objet fixe.

3. Système d'assistance à un utilisateur selon la revendication 1 ou la revendication 2, **caractérisé en ce que** la caractéristique (41) d'identification d'utilisateur est un mot de passe et/ou une caractéristique biométrique de l'utilisateur.

4. Système d'assistance à un utilisateur selon l'une des revendications 1 à 3, **caractérisé en ce que** le système d'assistance à un utilisateur est agencé pour émettre des instructions concernant le retraitement du produit médical sous forme d'informations visuelles et pour surveiller une exécution de ces instructions par évaluation des données d'image enregistrées par la caméra (16), le système d'assistance à un utilisateur étant notamment agencé pour émettre un message d'erreur en cas d'exécution erronée des instructions.

5. Système d'assistance à un utilisateur selon l'une des revendications 1 à 4, **caractérisé en ce qu'**il comprend une source de lumière (21) qui émet dans une plage de longueurs d'onde dans laquelle des impuretés médicales (44), en particulier du sang ou des protéines, sont excitées en fluorescence, la caméra (16) étant agencée pour acquérir des images dans une plage de longueurs d'onde dans laquelle se produit la fluorescence des impuretés médicales (44), le système d'assistance à un utilisateur étant en outre conçu pour générer des images fluorescentes (46), notamment pour amplifier des images fluorescentes (46), et pour les représenter sur la surface de projection (20).

6. Système d'assistance à un utilisateur selon l'une des revendications 1 à 5, **caractérisé en ce que** la navigation dans les informations visuelles affichées sur la surface de projection (20) est apte à être commandée par
- une détection des mouvements des yeux.
- une reconnaissance de gestes,
- une reconnaissance vocale et/ou
- un actionnement mécanique d'un élément de saisie sur le boîtier (18) des lunettes (14) à données.

7. Système d'assistance à un utilisateur selon l'une des revendications 1 à 6, **caractérisé en ce que** le système d'assistance à un utilisateur est agencé pour enregistrer et documenter les processus de retraitement en cours et achevés, le système d'assistance à un utilisateur comprenant en outre une mémoire de données externe (29) qui est agencée pour enregistrer les informations concernant les processus de retraitement en cours et achevés.

8. Système d'assistance à un utilisateur selon l'une des revendications 1 à 7, **caractérisé en ce que** le système d'assistance à un utilisateur est conçu pour associer à au moins une opération de retraitement un temps de retraitement prévu, et pour afficher sur la surface de projection (20) une durée restante du temps de retraitement prévu, le système d'assistance à un utilisateur étant notamment conçu pour afficher sur la surface de projection (20), après l'expiration du temps de retraitement prévu, des informations concernant une position d'un produit médical (5) dont le temps de retraitement prévu est écoulé.

9. Procédé de surveillance du retraitement de produits médicaux réutilisables (5), dans lequel une pluralité d'enregistrements (30a, 30b, 30c) de données de type pour différents types de produits médicaux réutilisables (5) et des informations (36) concernant des instructions pour le retraitement de ces types de produits médicaux sont mémorisés dans une mémoire de données (28) et dans chaque enregistrement (30a, 30b, 30c) de données de type sont présentes des informations (301a) concernant une caractéristique d'identification (6) d'un type de produit médical déterminé,
- des informations visuelles étant projetées sur une surface de projection (20) disposée dans le champ de vision d'un utilisateur de lunettes (14) à données,
- au moins une image du produit médical réutilisable (5) étant capturée au moyen d'une caméra (16), les données d'image de l'image étant comparées aux informations (301a) concernant les caractéristiques d'identification (6) dans les enregistrements (30a, 30b, 30c) de données de type, un type du produit médical réutilisable (5) étant identifié à l'aide d'une caractéristique d'identification (6) présente dans l'image et un profil de type (302a) associé à un enregistrement de données de type correspondant (30a, 30b, 30c) étant activé,
- en outre, une caractéristique (41) d'identification d'utilisateur est détectée, comparée avec des enregistrements (32a, 32b, 32c) de données d'utilisateur enregistrés sur la mémoire de données (238), qui comprennent respectivement des informations (321a) concernant au moins une caractéristique (41) d'identification d'utilisateur, et en cas de concordance, un profil d'utilisateur (322a) associé à l'enregistrement (32a, 32b, 32c) de données d'utilisateur est activé,
- une caractéristique (51, 53) d'identification de localisation étant en outre détectée, comparée avec des ensembles (34a, 34b, 34c) de données de localisation enregistrés sur la mémoire de données (28), qui comprennent chacun des informations (341a) concernant au moins une caractéristique (51, 53) d'identification de localisation, et un profil de localisation (342a) associé à l'ensemble de données de localisation (34a, 34b, 34c) étant activé en cas de concordance,
- **caractérisé en ce que**
la mémoire de données (238) comprend des informations (36) concernant des instructions pour le retraitement du produit médical (5), qui sont associées à au moins un profil type (302a), au moins un profil utilisateur (322a) et au moins un profil de localisation (342a), et dans lequel les instructions pour le retraitement du produit médical (5) associées au profil type actif (302a), au profil utilisateur actif (322a) et au profil de localisation actif (342a) sont affichées sous forme d'informations visuelles sur la surface de projection (20).

10. Procédé selon la revendication 9, **caractérisé en ce que** la caractéristique (51, 53) d'identification de localisation
- est stockée dans un transpondeur RFID ou NFC,
- est codé dans un code à barres ou un code DataMatrix et/ou
- consiste en une apparence extérieure d'au moins un objet fixe.

11. Procédé selon la revendication 9 ou la revendication 10, **caractérisé en ce que** la caractéristique (41) d'identification d'utilisateur est un mot de passe et/ou une caractéristique biométrique de l'utilisateur.

12. Procédé selon l'une des revendications 9 à 11, **caractérisé en ce que** des instructions concernant le retraitement du produit médical (5) sont émises sous forme d'informations visuelles et **en ce que** l'exécution d'au moins une de ces instructions est surveillée par l'exploitation des données d'image enregistrées par la caméra (16), un message d'erreur étant notamment émis en cas d'exécution erronée de l'instruction.

13. Procédé selon l'une des revendications 9 à 12, **caractérisé en ce qu'**au moyen d'une source lumineuse (21), on émet de la lumière dans une plage de longueurs d'onde dans laquelle des impuretés médicales (44), en particulier du sang ou des protéines, sont excitées en fluorescence, des données d'image étant enregistrées au moyen de la caméra (16) dans une plage de longueurs d'onde dans laquelle la fluorescence des impuretés médicales (44) se produit, des images fluorescentes (46) étant en outre générées, notamment amplifiées, et ces images fluorescentes (46) étant affichées sur la surface de projection (20).

14. Procédé selon l'une quelconque des revendications 9 à 13, **caractérisé en ce que** la navigation dans les informations visuelles affichées sur la surface de projection (20) est commandée par
- une détection des mouvements des yeux,
- une reconnaissance de gestes,
- une reconnaissance vocale, et/ou
- un actionnement mécanique d'un élément de saisie sur le boîtier (18) des lunettes (14) à données.

15. Procédé selon l'une des revendications 9 à 14, **caractérisé en ce que** les opérations de reconditionnement en cours et terminées sont enregistrées et documentées, les informations concernant les opérations de reconditionnement en cours et terminées étant enregistrées sur une mémoire de données externe (29).

16. Procédé selon l'une des revendications 9 à 15, **caractérisé en ce qu'**un temps de traitement prévu est associé à au moins une opération de traitement, la durée restante du temps de traitement prévu étant affichée sur la surface de projection (20), des informations concernant une position d'un produit médical (5) dont le temps de traitement prévu est écoulé étant notamment affichées sur la surface de projection (20) à l'expiration du temps de traitement prévu.

17. Programme d'ordinateur comprenant des moyens de code de programme qui amènent le dispositif selon l'une des revendications 1 à 8 à mettre en oeuvre les étapes de procédé selon l'une des revendications 9 à 16.
